# FASCICULE DE BREVET EUROPEEN

(11) **EP 1 901 735 B1**
(45) Date de publication et mention de la délivrance du brevet: **02.05.2012**
(21) Numéro de dépôt: 06778654.1
(22) Date de dépôt: 23.06.2006
(51) Int. Cl.: C07D 493/18, A61K 31/357

(54) **Composes de type polyphenols pour la préparation de compositions destinées à prevenir ou traiter des maladies impliquant une proliferation cellulaire anormale**
Verbindungen vom Polyphenol-typ zur Herrstellung von Zusammensetzungen zur Prävention oder Behandlung von Erkrankungen mit abnormaler Zellproliferation
Polyphenol type compounds for the manufacture of a composition for preventing or treating diseases involving abnormal cell proliferation

(30) Priorité: 01.07.2005 FR 0507051
(43) Date de publication de la demande: 26.03.2008
(73) Titulaire: Fluofarma, 33600 Pessac (FR); Université Bordeaux 1, 33405 Talence Cedex (FR)
(72) Inventeur: DEPIERRE, Gaëlle, F-33710 Saint Seurin de Bourg (FR); QUIDEAU, Stéphane, F-33400 Talence (FR); JOURDES, Michael, F-16110 Pranzac (FR); PARDON, Patrick, F-33140 Villenave d'Ornon (FR); POURQUIER, Philippe, F-33750 Beychac et Caillau (FR)
(74) Mandataire: Novagraaf Technologies
(86) Numéro de dépôt international: PCT/FR2006/001455
(87) Numéro de publication internationale: WO 2007/003741

(56) Documents cités:
- WO-A-01/21228
- KASHIWADA YOSHIKI, NONAKA GEN-ICHIRO, NISHIOKA ITSUO, CHANG JER-JANG, LEE KUO-HSIUNG: "Antitumor agents, 129. Tannins and related compounds as selective cytotoxic agents" JOURNAL OF NATURAL PRODUCTS, vol. 55, no. 8, 1992, pages 1033-1043, XP009064352
- PATENT ABSTRACTS OF JAPAN vol. 2003, no. 12, 5 décembre 2003 (2003-12-05) & JP 2004 300049 A (CCI CORP), 28 octobre 2004 (2004-10-28)
- QUIDEAU STEPHANE; JOURDES MICHAEL;LEFEUVRE DOROTHEE;MONTAUDON DANIÈLE;SAUCIER CEDRIC; GLORIES YVES;PARDON PATRICK;POURQUIER PHILIP: "The Chemistry of wine polyphenolic C-glycosidic ellagitannins targetting human topoisomerase II" CHEMISTRY A EUROPEAN JOURNAL, vol. 11, no. 22, novembre 2005 (2005-11), pages 6503-65013, XP009064322

## Description

La présente invention concerne des composés de type polyphénols, notamment à titre de médicament, l'utilisation de tels composés dans la préparation de compositions pharmaceutiques. L'invention concerne également des compositions comprenant des composés de ce type, ou encore l'utilisation de ce type de composés pour la préparation de compositions pro-apoptotiques. Les compositions peuvent notamment être destinées à prévenir ou à traiter des maladies impliquant une prolifération cellulaire anormale, par exemple le cancer.

Le cancer est une des causes de mortalité les plus importante et par conséquent un des problèmes de santé publique les plus graves dans le monde actuel. À ce titre, de nombreux médicaments ont été et sont développés, malheureusement ils ne permettent pas de traiter tous les cas avec succès. En particulier, il a été découvert des résistances à certains agents anticancéreux. Il est donc important de rechercher de nouveaux moyens thérapeutiques et de mettre en place de nouvelles stratégies de traitement.

Plusieurs agents antitumoraux sont des inhibiteurs de l'ADN topoisomérase II. Cependant, il est également connu que certains composés inhibant l'ADN topoisomérase II peuvent stimuler la prolifération de cellules cancéreuses à des concentrations subtoxiques. Ceci a notamment été décrit pour certains composés de la famille des anthracyclines par P. Vichi et T.R. Tritton, Cancer Res., 1989, 49(10), 2679-82, et par M.G. Thompson et J.A. Hickman, Eur. J. Cancer, 1991, (27)10, 1263-8.

L'activité antitumorale de certains tanins par leur effet cytotoxique sur des lignées cellulaires de cancer humain (cf. page 1035, 1^{er} paragraphes en particulier, que l'acutissimine A montre une cytotoxicité modérée et sélective contre les cellules de mélanome PRMI-7951 (cf. abstract) est décrit par KASHIWADA YOSHIKI, NONAKA GEN-ICHIRO, NISHIOKA ITSUO, CHANG JER-JANG, LEE KUO-HSIUNG: "Antitumor agents, 129. Tannins and related compounds as selective cytotoxic agents" JOURNAL OF NATURAL PRODUCTS, vol. 55, no. 8, 1992, pages 1033-1043, XP009064352

Des composés ayant des propriétés antiallergiques, sans pour autant présenter d'effets secondaires, ainsi que des denrées alimentaires les contenant sont décrits dans PATENT ABSTRACTS OF JAPAN vol. 2003, no. 12, 5 décembre 2003

WO 01/21228 A (BAXTER INTERNATIONAL INC; TASIAUX, NICOLE; DELMOTTE, YVES) 29 mars 2001 (2001-03-29) décrit une valve cardiaque fabriquée en un tissu biologique ou biocompatible ayant une resistance à la calcification, comprenant un agent actif contre la calcification (cf. page 54, lignes 17-36). Parmi les agents actifs contre la calcification sont cités la vescaline (cf. page 6, composé 27 et page page 54, ligne 33) et la castaline (cf. page 11).

Certains composés polyphénoliques de type C-glycosides d'ellagitannins sont des inhibiteurs de l'ADN topoisomérase II. Parmi ceux-ci, certains composés de la famille des ellagitannins C-glycosidiques comprenant un radical nonahydroxyterphénoyl (NHTP) polyphénolique et de leurs dérivés, par exemple la vescalagine, la castalagine, l'acutissimine A et B, et l'épiacutissamine A et B, présentent un effet de stimulation de la prolifération de cellules cancéreuses à des doses subtoxiques.

Il est clair que cet effet est particulièrement gênant dans le cadre de l'utilisation de ces composés dans la lutte contre des maladies impliquant une prolifération cellulaire anormale.

Ainsi un des but de l'invention est de fournir de nouveaux composés inhibiteurs de l'ADN topoisomérase II présentant une activité anticancéreuse améliorée, notamment reliée à une activité pro-apoptotique et en particulier ne favorisant pas la prolifération des cellules cancéreuses à des doses subtoxiques.

Les inventeurs ont maintenant découvert que les composés tels que définis ci-dessous sont des inhibiteurs d'ADN topoisomérase II et présentent une activité anticancéreuse, notamment reliée à une activité pro-apoptotique et n'augmentent pas ou peu la prolifération des cellules cancéreuses, en particulier à des doses subtoxiques.

Ainsi, l'invention a pour objet des composés répondant à la formule (I) suivante, ou ses sels pharmaceutiquement acceptables, à titre de médicament : 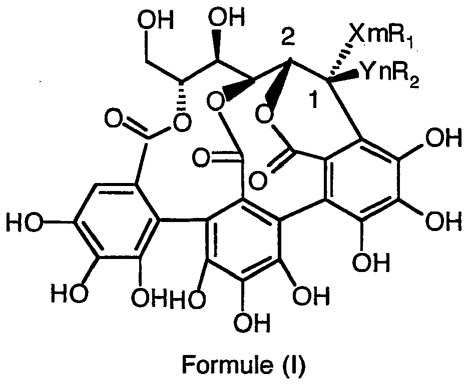 dans laquelle :
- XₘR₁ et YₙR₂, identiques ou différents, sont présents, ou
- l'un de XₘR₁ ou YₙR₂ est absent, et dans ce cas la liaison entre C₁ et C₂ est une double liaison, ou
- XₘR₁ et YₙR₂ ensemble représentent un groupe A et dans ce cas la liaison entre C₁ et A est une double liaison,
   où :
- R₁ et R₂ représentent indépendamment l'un de l'autre un atome d'hydrogène ; un atome d'halogène ; un radical alkyle, alcène ou alcyne, comprenant de 1 à 18 atomes de carbone, éventuellement substitué, par un ou plusieurs groupements amino, acide, dérivé d'acide, alkoxy, aryle ou hydroxy ; un radical aryle, éventuellement substitué par un ou plusieurs groupements amino, acide, dérivé d'acide, alkoxy, aryle ou hydroxy ; un radical arylalkyle ou alkylaryle, éventuellement substitué par un ou plusieurs groupements amino, acide, dérivé d'acide, alkoxy, aryle ou hydroxy ; ou une fonction nitro, ;
   ou R₁ et R₂ forment ensemble un cycle ou un hétérocycle, éventuellement substitué,
- m est un nombre entier choisi parmi 0 et 1, n est un nombre entier choisi parmi 0 et 1,
- X et Y représentent indépendamment l'un de l'autre -O-, -OC(=O)-, -OC(=O)O-, -OC(=O)N-, -NR₃-, -NR₃C(=O)-, -NR₃C(=O)O-, -NR₃C(O)NR₃-, -C(=O)-, -C(=O)O--S(O)_{q}-, ou -P(O)ᵣ-,
- q est un nombre entier choisi parmi 0, 1, 2 et 3, r est un nombre entier choisi parmi 0, 1, 2 et 3,
- R₃ représente un atome d'hydrogène ; un radical alkyle, alcène ou alcyne, comprenant de 1 à 18 carbones, éventuellement substitué par un ou plusieurs groupements amino, acide, dérivé d'acide, alkoxy, aryle ou hydroxy ; un radical aryle, éventuellement substitué par un ou plusieurs groupements amino, acide, dérivé d'acide, alkoxy, aryle ou hydroxy ; un radical arylalkyle ou alkylaryle éventuellement substitué par un ou plusieurs groupements amino, acide, dérivé d'acide, alkoxy, aryle ou hydroxy ; ou un radical alcoxy, éventuellement substitué par un radical aryle ;
- A représente O, S, NOR₅, NR₆ ou CR₅R₆, où R₅ et R₆ représentent indépendamment l'un de l'autre un atome d'hydrogène ; un radical alkyle, alcène ou alcyne, comprenant de 1 à 18 atomes de carbone, éventuellement substitué par un ou plusieurs groupements amino, acide, dérivé d'acide, alkoxy, aryle ou hydroxy ; un radical arylalkyle ou alkylaryle éventuellement substitué par un ou plusieurs groupements amino, acide, dérivé d'acide, alkoxy, aryle ou hydroxy ; ou un radical alcoxy, éventuellement substitué par un radical aryle.

Bien entendu, les composés selon l'invention peuvent également être des isomères optiques et géométriques, notamment au niveau du carbone 1, des composés définis ci-dessus, ainsi que leurs mélanges racémiques.

Des composés préférés selon l'invention répondent à la formule (I) suivante, ou un de ses sels pharmaceutiquement acceptable : 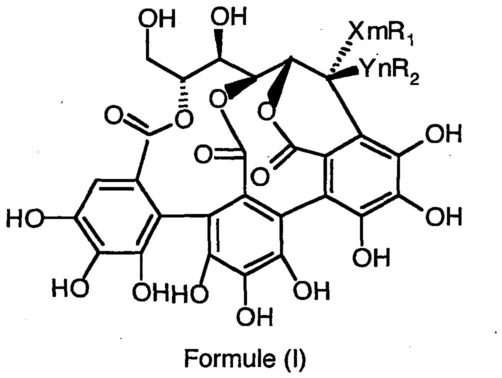 dans laquelle :
- R₁ et R₂ représentent indépendamment l'un de l'autre un atome d'hydrogène ; un atome d'halogène ; un radical alkyle, alcène ou alcyne, comprenant de 1 à 18 atomes de carbone, éventuellement substitué par un ou plusieurs groupements amino, acide, dérivé d'acide, alkoxy, aryle ou hydroxy ; un radical aryle, éventuellement substitué, par un ou plusieurs groupements amino, acide, dérivé d'acide, alkoxy, aryle ou hydroxy ; un radical arylalkyle ou alkylaryle, éventuellement substitué par un ou plusieurs groupements amino, acide, dérivé d'acide, alkoxy, aryle ou hydroxy ; ou une fonction nitro,
ou R₁ et R₂ forment ensemble un cycle ou un hétérocycle, éventuellement substitué,
- m est un nombre entier choisi parmi 0 et 1, n est un nombre entier choisi parmi 0 et 1,
- X et Y représentent indépendamment l'un de l'autre -O-, -OC(=O)-, -OC(=O)O-, -OC(=O)N-, -NR₃-, -NR₃C(=O)**-,** -NR₃C(=O)O-, -NR₃C(O)NR₃-, -C(=O)-, -C(=O)O-, -S(O)_{q}-, -P(O)ᵣ-,
- q est un nombre entier choisi parmi 0, 1, 2 et 3, r est un nombre entier choisi parmi 0, 1, 2 et 3,
- R₃ représente un atome d'hydrogène ; un radical alkyle, alcène ou alcyne, comprenant de 1 à 18 carbones, éventuellement substitué par un ou plusieurs groupements amino, acide, dérivé d'acide, alkoxy, aryle ou hydroxy ; un radical aryle, éventuellement substitué par un ou plusieurs groupements amino, acide, dérivé d'acide, alkoxy, aryle ou hydroxy ; un radical arylalkyle ou alkylaryle, éventuellement substitué par un ou plusieurs groupements amino, acide, dérivé d'acide, alkoxy, aryle ou hydroxy ; ou un radical alcoxy, éventuellement substitué par un radical aryle.

D'autres composés préférés selon l'invention répondent à la formule (II) suivante ou un de ses sels pharmaceutiquement acceptable : 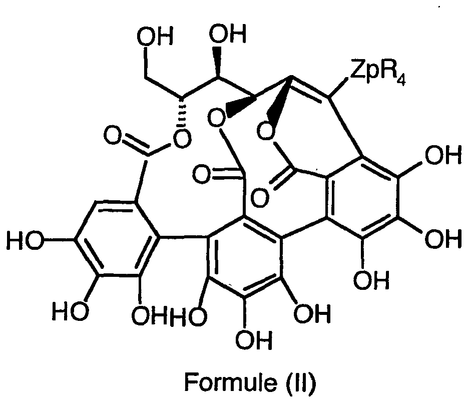 dans laquelle :
- p représente un nombre entier choisi parmi 0 et 1,
- Z représente -O-, -OC(=O)-, -OC(=O)O-, - OC(=O)N-, -NR₃-, -NR₃C(=O)-, -NR₃C(=O)O-, -NR₃C(O)NR₃-, - C(=O)-, -C(=O)O-, -S(O)q- ou -P(O)ᵣ-,
- q est un nombre entier choisi parmi 0, 1, 2 et 3, r est un nombre entier choisi parmi 0, 1, 2 et 3,
- R₃ est tel que défini ci-dessus,
- R₄ représente un atome d'hydrogène ; un atome d'halogène ; un radical alkyle, alcène ou alcyne, comprenant de 1 à 18 atomes de carbone, éventuellement substitué par un ou plusieurs groupements amino, acide, dérivé d'acide, alkoxy, aryle ou hydroxy ; un radical aryle, éventuellement substitué par un ou plusieurs groupements amino, acide, dérivé d'acide, alkoxy, aryle ou hydroxy ; un radical arylalkyle ou alkylaryle, éventuellement substitué par un ou plusieurs groupements amino, acide, dérivé d'acide, alkoxy, aryle ou hydroxy ; un radical alcoxy, éventuellement substitué par un radical aryle, ou une fonction nitro.

Encore d'autres composés selon l'invention répondent à la formule (III) suivante, ou un de ses sels pharmaceutiquement acceptable : 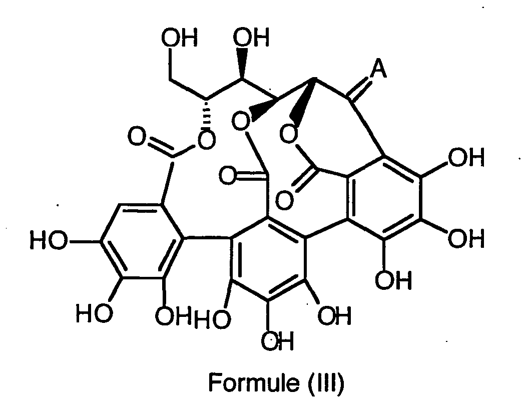 dans laquelle :
- A représente O, S, NOR₅, NR₆ ou CR₅R₆,
- R₅ représente un atome d'hydrogène ; un radical alkyle, alcène ou alcyne, comprenant de 1 à 18 atomes de carbone, éventuellement substitué par un ou plusieurs groupements amino, acide, dérivé d'acide, alkoxy, aryle ou hydroxy ; un radical aryle, éventuellement substitué par un ou plusieurs groupements amino, acide, dérivé d'acide, alkoxy, aryle ou hydroxy ; un radical arylalkyle ou alkylaryle, éventuellement substitué par un ou plusieurs groupements amino, acide, dérivé d'acide, alkoxy, aryle ou hydroxy ; ou un radical alcoxy, éventuellement substitué par un radical aryle, et
- R₆ représente un radical alkyle, alcène ou alcyne, comprenant de 1 à 18 atomes de carbone, éventuellement substitué par un ou plusieurs groupements amino, acide, dérivé d'acide, alkoxy, aryle ou hydroxy ; un radical aryle, éventuellement substitué par un ou plusieurs groupements amino, acide, dérivé d'acide, alkoxy, aryle ou hydroxy ; un radical arylalkyle ou alkylaryle, éventuellement substitué par un ou plusieurs groupements amino, acide, dérivé d'acide, alkoxy, aryle ou hydroxy ; ou un radical alcoxy, éventuellement substitué par un radical aryle.

Par atome d'halogène, on désigne un atome choisi parmi le fluor, le chlore, le brome et l'iode.

Les radicaux alkyles, alcènes ou alcynes peuvent comprendre de 1 à 18 atomes de carbones, notamment de 1 à 12 atomes de carbones, et en particulier de 1 à 6 atomes de carbone.

Les radicaux alcènes peuvent comprendre une ou plusieurs double liaison.

Les radicaux alcynes peuvent comprendre une ou plusieurs triple liaison.

Les radicaux aryles peuvent comprendre de 6 à 14 atomes de carbone et notamment de 6 à 10 atomes de carbone.

Les radicaux arylalkyles et alkylaryles peuvent comprendre de 7 à 25 atomes de carbones, notamment de 7 à 20 atomes de carbone et en particulier de 7 à 15 atomes de carbone. Tout particulièrement le radical alkylaryle peut représenter un benzyle.

Lorsque R₁ et R₂ forment ensemble un cycle ou un hétérocycle, celui-ci peut présenter de 4 à 10 chaînons, et notamment de 6 à 8 chaînons.

Un hétérocycle comprend dans son cycle outre des atomes de carbones au moins un hétéroatome, notamment choisi parmi l'oxygène, l'azote et le soufre.

Un premier groupe de composés préférés selon l'invention sont ceux de formule (I) dans laquelle m est 0 et R₁ représente un atome d'hydrogène, n est 1, Y représente -O- et R₂ représente un groupement choisi dans le groupe comprenant un atome d'hydrogène et un radical alkyle comprenant de 1 à 18 atomes de carbone. En particulier R₂ peut représenter un groupement choisi dans le groupe comprenant un atome d'hydrogène et un radical alkyle comprenant de 1 à 6 atomes de carbone, et notamment un groupement choisi dans le groupe comprenant un atome d'hydrogène, le radical méthyle, éthyle, propyle, butyle, pentyle et hexyle.

Un autre groupe de composés préférés selon l'invention sont ceux de formule (I) dans laquelle m est 1, X représente -O-, R₁ représente un groupement choisi dans le groupe comprenant un atome d'hydrogène et un radical alkyle comprenant de 1 à 18 atomes de carbone, n est 0, et R₂ représente un atome d'hydrogène. En particulier R₁ peut représenter un groupement choisi dans le groupe comprenant un atome d'hydrogène et un radical alkyle comprenant de 1 à 6 atomes de carbone, et notamment un groupement choisi dans le groupe comprenant un atome d'hydrogène, le radical méthyle, éthyle, propyle, butyle, pentyle et hexyle.

Encore un autre groupe de composés préférés selon l'invention sont ceux de formule (II) dans laquelle p est 0 et R₄ représente un groupement choisi dans le groupe comprenant un atome d'hydrogène et un radical alkyle comprenant de 1 à 18 atomes de carbone. En particulier R₄ peut représenter un groupement choisi dans le groupe comprenant un atome d'hydrogène et un radical alkyle comprenant de 1 à 6 atomes de carbone, et notamment un groupement choisi dans le groupe comprenant un atome d'hydrogène, le radical méthyle, éthyle, propyle, butyle, pentyle et hexyle.

Un autre groupe de composés préférés selon l'invention sont ceux de formule (III) dans laquelle A représente O.

L'invention envisage à titre de composés spécifiques :
- la Vescaline, correspondant à la formule (I) dans laquelle m est 0, R₁ représente un atome d'hydrogène, n est 1, Y représente -O-, et R₂ représente un atome d'hydrogène,
- la Castaline, correspondant à la formule (I) dans laquelle m est 1, X représente -O-, R₁ représente un atome d'hydrogène, n est 0, et R₂ représente un atome d'hydrogène, et
- le Vescalène correspondant à la formule (II) dans laquelle p est 0 et R₄ représente un atome d'hydrogène.

Les composés de formule (I), (II) ou (III) peuvent le cas échéant être sous forme solvatée, de sel, ou d'autres dérivées physiologiquement acceptables. Les sels et les solvants qui sont acceptables pour une utilisation pharmaceutique sont généralement ceux dans lesquels le contre ion ou le solvant associé est pharmaceutiquement acceptable.

Les sels utilisables peuvent être des acides ou des bases organiques ou minérales. Parmi les sels d'addition acides acceptables, on peut citer ceux formés à partir d'acide chlorhydrique, bromhydrique, sulfurique, citrique, tartrique, phosphorique, lactique, pyruvique, acétique, trifluoroacétique, phénylacétique, triphénylacétique.

On peut également citer parmi les sels basiques acceptables, les sels de métaux alcalins, tels que le sodium ou le potassium, les sels de métaux alcalino-terreux, tels que le calcium et le magnésium, et les sels formés à partir de bases organiques, tels que les amines mono-, di- ou tri-substituées.

L'invention se rapporte également à des compositions pharmaceutiques contenant à titre d'agent actif au moins un composé tel que défini précédemment.

Dans la composition pharmaceutique, les composés sont employés en quantité efficace. Celle-ci sera déterminée par l'homme du métier, selon différents paramètres, en particulier par rapport à la substance utilisée, l'age, le poids, et l'état physique du patient, le mode d'administration, et le régime requis. Un médecin sera à même de déterminer le mode d'administration et le dosage pour chaque patient.

La composition pharmaceutique peut être administrée sous toute forme, topique ou systémique, notamment sous forme parentérale ou entérale.

Lorsque la composition ou le médicament sont administrés par voie entérale, elle peut se présenter sous forme de comprimés, de gélules, de dragées, de sirops, de suspensions, de solutions, de poudres, de granulés, d'émulsions, de microsphères.

Dans le cas d'une administration par voie parentérale, la composition peut se présenter sous forme de solutions ou de suspensions pour perfusion ou pour injection.

La composition comprend au moins un support pharmaceutiquement acceptable.

Elle peut également comprendre au moins un additif, choisi notamment parmi les agents de couleur, de saveur, et les conservateurs. Bien entendu, l'homme du métier veillera à choisir le ou les additif(s) de manière à ce que les propriétés avantageuses attachées intrinsèquement à l'invention ne soient pas, ou substantiellement pas, altérées par l'addition envisagée.

Selon une variante particulière, la composition comprend de l'éthanol dans une teneur inférieure à 10 % en poids, notamment inférieure à 8 % en poids, en particulier inférieure à 6 % en poids, plus particulièrement inférieure à 4 % en poids, et encore plus particulièrement inférieure à 2 % en poids par rapport au poids total de la composition, tout particulièrement ladite composition est dépourvue d'éthanol.

Selon un autre aspect, l'invention a encore pour objet l'utilisation d'au moins un composé de formule (I), (II) ou (III) ou d'un de leurs sels pharmaceutiquement acceptable, dans la préparation d'une composition destinée à prévenir ou à traiter au moins une maladie impliquant une prolifération cellulaire anormale.

Ladite composition peut être destinée à la médecine humaine et/ou vétérinaire, et en particulier elle peut être destinée à traiter ou à prévenir au moins un cancer choisi parmi le cancer du pancréas, les cancers de l'oro-pharynx, le cancer de l'estomac, le cancer de l'oesophage, le cancer du colon et rectal, le cancer du cerveau, notamment les gliomes, le cancer des ovaires, le cancer du foie, le cancer du rein, le cancer du larynx, le cancer de la thyroïde, le cancer du poumon, le cancer des os, les myélomes multiples, les mésothéliomes et les mélanomes, le cancer de la peau, le cancer du sein, le cancer de la prostate, le cancer de la vessie, le cancer de l'utérus, le cancer des testicules, les lymphomes non-Hodgkinien, la leucémie, la maladie de Hodgkin, et des cancers des tissus mous, ainsi que des localisations secondaires métastatiques des cancers cités précédemment.

Par « prolifération anormale » on entend une prolifération qui est indépendante des mécanismes de régulations normaux, par exemple l'arrêt de prolifération cellulaire dû à la mise en jeu de l'apoptose (mort cellulaire programmée).

Selon un autre aspect, l'invention a pour objet l'utilisation d'au moins un composé de formule (I), (II) ou (III) ou d'un de leurs sels pour la préparation d'une composition pro-apoptotique.

D'autres avantages et caractéristiques de l'invention apparaîtront des exemples qui suivent concernant l'activité pro-apoptotique et dans lesquels il sera fait références aux dessins en annexe où :
- La figure 1 représente l'effet de la Castaline à différentes concentrations quant à l'apoptose à 24h et 48h et à la prolifération cellulaire à 24h et 48h.
- La figure 2 représente l'effet du Vescalène à différentes concentrations quant à l'apoptose à 24h et 48h et à la prolifération cellulaire à 24h et 48h.
- La figure 3 représente l'effet de la Castalagine à différentes concentrations quant à l'apoptose à 24h et 48h et à la prolifération cellulaire à 24h et 48h.
- La figure 4 représente l'effet de l'Acutissimine A à différentes concentrations quant à l'apoptose à 24h et 48h et à la prolifération cellulaire à 24h et 48h.

Les exemples suivants sont donnés à titre indicatif et sans aucun caractère limitatif de l'invention.

### EXEMPLES

### Exemple 1 : Activité pro-apoptotique et effet sur la prolifération cellulaire de la Castaline (encore appelée composé n°030501-07)

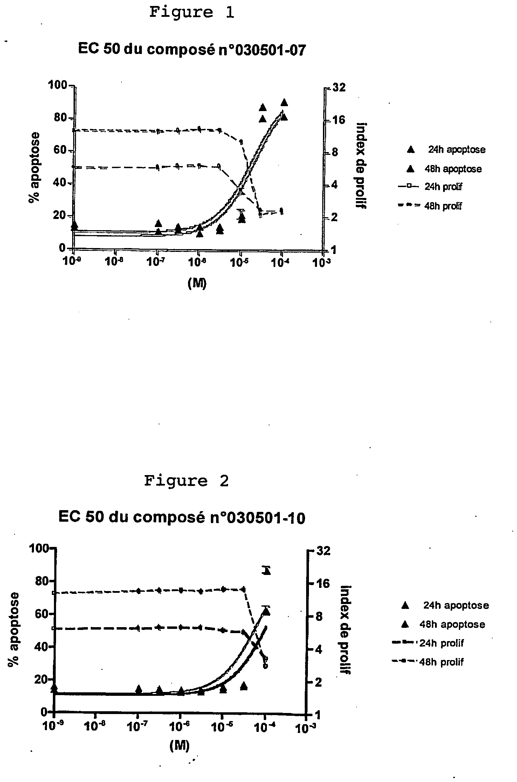

La figure 1 montre que la Castaline présente une activité pro-apoptotique et n'augmente pas de manière sensible la prolifération cellulaire à des doses subtoxiques.

### Exemple 2 : Activité pro-apoptoticrue et effet sur la prolifération cellulaire du Vescalène (encore appelé composé n°030501-10)

La figure 2 montre que le Vescalène présente une activité pro-apoptotique sans augmenter sensiblement la prolifération cellulaire à des doses subtoxiques.

### Exemple comparatif 1 : Activité ro-apoptotique et effet sur la prolifération cellulaire de la Castalagine (encore appelé composé n°030501-01)

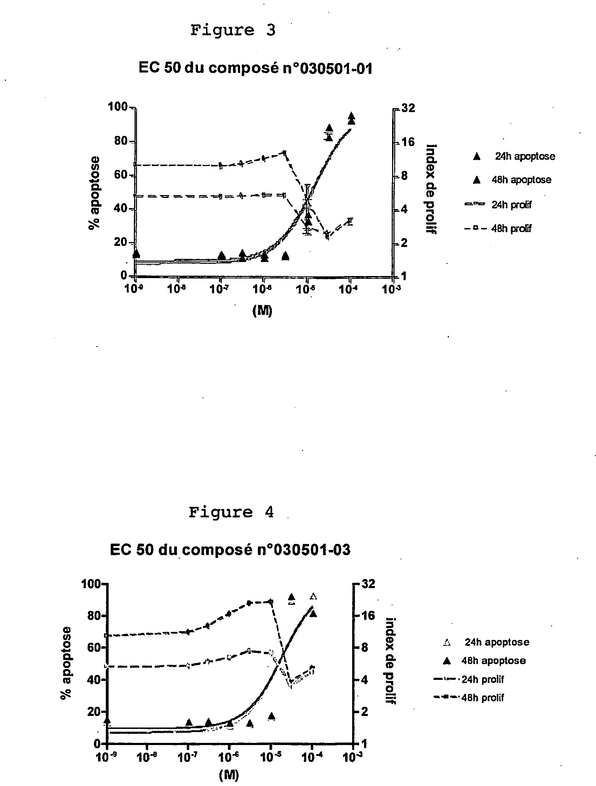

La figure 3 montre que la Castalagine engendre une augmentation de la prolifération cellulaire à des doses subtoxiques, notamment dans la zone 1 à 10 µM.

### Exemple comparatif 2 : Activité pro-apoptotique et effet sur la prolifération cellulaire de l'Acutissimine A (encore appelé composé n-030501-03)

La figure 4 montre que l'Acutissimine A augmente la prolifération cellulaire à des doses subtoxiques, en particulier dans la zone 1 à 10 µM.

### Exemple 3 Inhibition de la l'ADN topoisomérase II

| Composé (concentration) | inhibition de la décaténation (%) |
|---|---|
| Vescaline (1 µm) | 95,5 |
| Vescaline (10 µm) | 100 |
| Castaline (1 µm) | 87,9 |
| Castaline (10 µm) | 67,0 |
| Vescalène (1 µm) | 78,8 |
| Vescalène (10 µm) | 97,3 |

Ces résultats montrent l'activité inhibitrice sur l'ADN topoisomérase II de composés selon l'invention.

## Revendications

1. Utilisation d'un composé répondant à la formule (I) suivante, ou un de ses sels pharmaceutiquement acceptable, dans la préparation une composition destinée à prévenir ou à traiter au moins une maladie impliquant une prolifération cellulaire anormale : 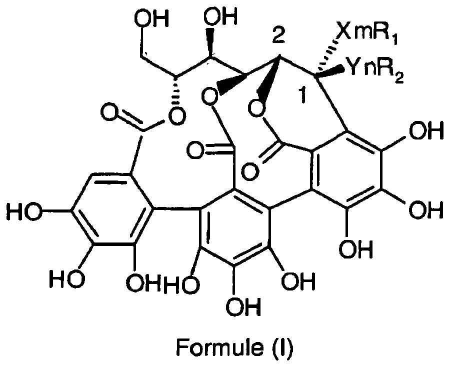 dans laquelle :
- XₘR₁ et YₙR₂, identiques ou différents, sont présents, ou
- l'un de XₘR₁ ou YₙR₂ est absent, et dans ce cas la liaison entre C₁ et C₂ est une double liaison,
- XₘR₁ et YₙR₂ ensemble représentent un groupe A et dans ce cas la liaison entre C₁ et A est une double liaison,
où :
- R₁ et R₂ représentent indépendamment l'un de l'autre un atome d'hydrogène ; un atome d'halogène ; un radical alkyle, alcène ou alcyne, comprenant de 1 à 18 atomes de carbone, éventuellement substitué par un ou plusieurs groupements amino, acide, dérivé d'acide, alkoxy, aryle ou hydroxy ; un radical aryle, éventuellement substitué par un ou plusieurs groupements amino, acide, dérivé d'acide, alkoxy, aryle ou hydroxy ; un radical arylalkyle ou alkylaryle, éventuellement substitué par un ou plusieurs groupements amino, acide, dérivé d'acide, alkoxy, aryle ou hydroxy ou une onction nitro ;
ou R₁ et R₂ forment ensemble un cycle ou un hétérocycle éventuellement substitué,
- m est un nombre entier choisi parmi 0 et 1, n est un nombre entier choisi parmi 0 et 1,
- X et Y représentent indépendamment l'un de l'autre -O-, -OC(=O)-, -OC(=O)O-, -OC(=O)N-, -NR₃-, -NR₃C(=O)-, -NR₃C(=O)O-, -NR₃C(O)NR₃-, -C(=O)-, -C(=O)O-, -S(O)_{q}-, ou -P(O)ᵣ-,
- q est un nombre entier choisi parmi 0, 1, 2 et 3, r est un nombre entier choisi parmi 0, 1, 2 et 3,
- R₃ représente un atome d'hydrogène ; un radical alkyle, alcène ou alcyne, comprenant de 1 à 18 carbones, éventuellement substitué par un ou plusieurs groupements amino, acide, dérivé d'acide, alkoxy, aryle ou hydroxy ; un radical aryle, éventuellement substitué par un ou plusieurs groupements amino, acide, dérivé d'acide, alkoxy, aryle ou hydroxy ; un radical arylalkyle ou alkylaryle, éventuellement substitué par un ou plusieurs groupements amino, acide, dérivé d'acide, alkoxy, aryl ou hydroxy ; ou un radical alcoxy, éventuellement substitué par un radical aryle ;
- A représente O, S, NOR₅, NR₆ ou CR₅R₆, où R₅ et R₆ représentent indépendamment l'un de l'autre un atome d'hydrogène ; un radical alkyle, alcène ou alcyne, comprenant de 1 à 18 atomes de carbone, éventuellement substitué par un ou plusieurs groupements amino, acide, dérivé d'acide, alkoxy, aryle ou hydroxy ; un radical arylalkyle ou alkylaryle éventuellement substitué par un ou plusieurs groupements amino, acide, dérivé d'acide, alkoxy, aryle ou hydroxy ; ou un radical alcoxy, éventuellement substitué par un radical aryle.

2. Utilisation selon la revendication 1, **caracterisee en ce que** ledit composé répond à la formule (I) suivante : 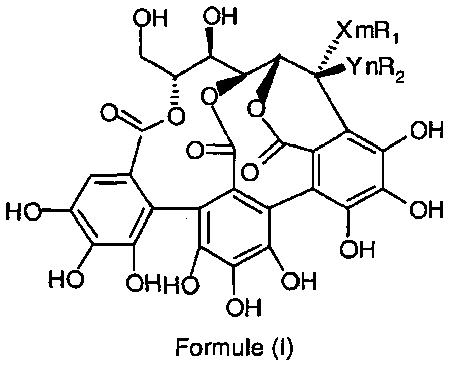 dans laquelle :
- R₁ et R₂ représentent indépendamment l'un de l'autre un atome d'hydrogène ; un atome d'halogène ; un radical alkyle, alcène ou alcyne, comprenant de 1 à 18 atomes de carbone, éventuellement substitué, par un ou plusieurs groupements amino, acide, dérivé d'acide, alkoxy, aryle ou hydroxy ; un radical aryle, éventuellement substitué par un ou plusieurs groupements amino, acide, dérivé d'acide, alkoxy, aryle ou hydroxy ; un radical arylalkyle ou alkylaryle, éventuellement substitué par un ou plusieurs groupements amino, acide, dérivé d'acide, alkoxy, aryle ou hydroxy ; ou une fonction nitro ;
ou R₁ et R₂ forment ensemble un cycle ou un hétérocycle éventuellement substitué,
- m est un nombre entier choisi parmi 0 et 1, n est un nombre entier choisi parmi 0 et 1,
- X et Y représentent indépendamment l'un de l'autre -O-, -OC(=O)-, -OC(=O)O-, -OC(=O)N-, -NR₃-, -NR₃C(=O)-, -NR₃C(=O)O-, -NR₃C(O)NR₃-, -C(=O)-, -C(=O)O-, -S(O)_{q}-, ou -P(O)ᵣ-,
- q est un nombre entier choisi parmi 0, 1, 2 et 3, r est un nombre entier choisi parmi 0, 1, 2 et 3,
- R₃ représente un atome d'hydrogène ; un radical alkyle, alcène ou alcyne, comprenant de 1 à 18 carbones, éventuellement substitué par un ou plusieurs groupements amino, acide, dérivé d'acide, alkoxy, aryle ou hydroxy ; un radical aryle, éventuellement substitué par un ou plusieurs groupements amino, acide, dérivé d'acide, alkoxy, aryle ou hydroxy ; un radical arylalkyle ou alkylaryle, éventuellement substitué par un ou plusieurs groupements amino, acide, dérivé d'acide, alkoxy, aryle ou hydroxy ; ou un radical alcoxy, éventuellement substitué par un radical aryle.

3. Utilisation selon la revendication 2, **caractérisée en ce que** ledit composé répond à la formule (I) dans laquelle m est 0 et R₁ représente un atome d'hydrogène, n est 1, Y représente -O- et R₂ représente un groupement choisi dans le groupe comprenant un atome d'hydrogène et un radical alkyle comprenant de 1 à 18 atomes de carbone.

4. Utilisation selon la revendication 2, **caractérisée en ce que** ledit composé répond à la formule (I) dans laquelle m est 1, X représente -O-, R₁ représente un groupement choisi dans le groupe comprenant un atome d'hydrogène et un radical alkyle comprenant de 1 à 18 atomes de carbone, n est 0, et R₂ représente un atome d'hydrogène.

5. Utilisation selon la revendication 1, **caractérisée en ce que** ledit composé répond à la formule (II) suivante : 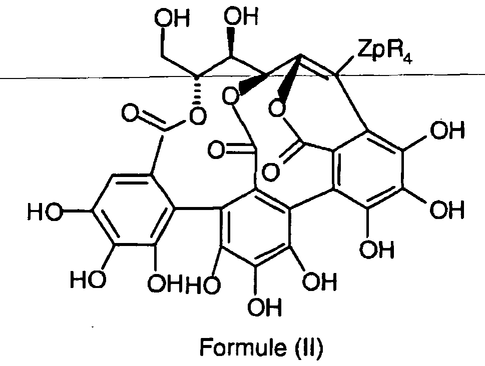 dans laquelle :
- p représente un nombre entier choisi parmi 0 et 1,
- Z représente -O-, -OC(=O)-, -OC(=O)O-, - OC(=O)N-, -NR₃-, -NR₃C(=O)-, -NR₃C(=O)O-, -NR₃C(O)NR₃-, - C(=O)-, -C(=O)O-, -S(O)_{q}-, ou -P(O)ᵣ-,
- q est un nombre entier choisi parmi 0, 1, 2 et 3, r est un nombre entier choisi parmi 0, 1, 2 et 3,
- R₃ représente un atome d'hydrogène ; un radical alkyle, alcène ou alcyne, comprenant de 1 à 18 carbones, éventuellement substitué par un ou plusieurs groupements amino, acide, dérivé d'acide, alkoxy, aryle ou hydroxy ; un radical arylalkyle ou alkyaryle, éventuellement substitué par un ou plusieurs groupements amino, acide, dérivé d'acide, alkoxy, aryle ou hydroxy ; ou un radical alcoxy, éventuellement substitué par un radical aryle, et
- R₄ représente un atome d'hydrogène ; un atome d'halogène ; un radical alkyle, alcène ou alcyne, comprenant de 1 à 18 atomes de carbone, éventuellement substitué par un ou plusieurs groupements amino, acide, dérivé d'acide, alkoxy, aryle ou hydroxy ; un radical aryle, éventuellement substitué par un ou plusieurs groupements amino, acide, dérivé d'acide, alkoxy, aryle ou hydroxy ; un radical arylalkyle ou alkyaryle, eventuellement substitué par un ou plusieurs groupements amino, acide, dérivé d'acide, alkoxy, aryle ou hydroxy ; un radical alcoxy, éventuellement substitué par un radical aryle ; ou une fonction nitro.

6. Utilisation selon la revendication 5, **caractérisée en ce que** ledit composé répond à la formule (II) dans laquelle p est 0 et R₄ représente un groupement choisi dans le groupe comprenant un atome d'hydrogène et un radical alkyle comprenant de 1 à 18 atomes de carbone.

7. Utilisation selon la revendication 1, **caractérisée en ce que** ledit composé répond à la formule (III) suivante : 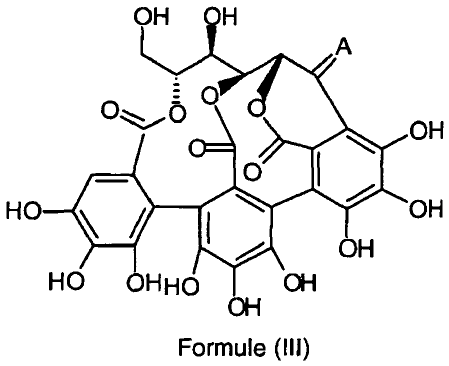 dans laquelle :
- A représente O, S, NOR₅, NR₆ ou CR₅R₆,
- R₅ représente un atome d'hydrogène ; un radical alkyle, alcène ou alcyne comprenant de 1 à 18 atomes de carbone éventuellement substitué par un ou plusieurs groupements amino, acide, dérivé d'acide, alkoxy, aryle ou hydroxy ; un radical aryle, éventuellement substitué par un ou plusieurs groupements amino, acide, dérivé d'acide, alkoxy, aryle ou hydroxy ; un radical arylalkyle ou alkylaryle, éventuellement substitué par un ou plusieurs groupements amino, acide, dérivé d'acide, alkoxy, aryle ou hydroxy ; ou un radical alcoxy, éventuellement substitué par un radical aryle, et
- R₆ représente un radical alkyle, alcène ou alcyne, comprenant de 1 à 18 atomes de carbone éventuellement substitué par un ou plusieurs groupements amino, acide, dérivé d'acide, alkoxy, aryle ou hydroxy ; un radical aryle, éventuellement substitué par un ou plusieurs groupements amino, acide, dérivé d'acide, alkoxy, aryle ou hydroxy ; un radical arylalkyle ou alkylaryle, éventuellement substitué par un ou plusieurs groupements amino, acide, dérivé d'acide, alkoxy, aryle ou hydroxy ; ou un radical alcoxy, éventuellement substitué par un radical aryle.

8. Utilisation selon la revendication 1, **caractérisée en ce que** ledit composé répond à la formule (III) dans laquelle A représente O.

9. Utilisation selon l'une quelconque des revendications 1 à 8 dans laquelle ladite maladie impliquant une prolifération cellulaire anormale est le cancer.

10. Utilisation selon la revendication 9, dans laquelle ledit cancer est un cancer choisi parmi le cancer du pancréas, les cancers de l'oropharynx, le cancer de l'estomac, le cancer de l'oesophage, le cancer du colon et rectal, le cancer du cerveau, notamment les gliomes, le cancer des ovaires, le cancer du foie, le cancer du rein, le cancer du larynx, le cancer de la thyroïde, le cancer du poumon, le cancer des os, les myélomes multiples, les mésothéliomes et les mélanomes, le cancer de la peau, le cancer du sein, le cancer de la prostate, le cancer de la vessie, le cancer de l'utérus, le cancer des testicules, es lymphomes non-Hodgkinien, la leucémie, la maladie de Hodgkin, et des cancers des tissus mous, ainsi que des localisations secondaires métastatiques des cancers cités précédemment.

11. Utilisation selon l'une quelconque des revendications 1 à 8, dans laquelle ladite composition destinée à prévenir ou à traiter au moins une maladie impliquant une prolifération cellulaire anormale est une composition pro-apoptotique.

12. Composé tel que défini selon l'une quelconque des revendications 1 à 8, pour la prévention ou le traitement d'au moins une maladie impliquant une prolifération cellulaire anormale.

13. Composé selon la revendication 12, **caractérisé en ce que** ladite maladie impliquant une prolifération cellulaire anormale est le cancer.

14. Composé selon la revendication 13, **caractérisé en ce que** ledit cancer est un cancer choisi parmi le cancer du pancréas, les cancers de l'oropharynx, le cancer de l'estomac, le cancer de l'oesophage, le cancer du colon et rectal, le cancer du cerveau, notamment les gliomes, le cancer des ovaires, le cancer du foie, le cancer du rein, le cancer du larynx, le cancer de la thyroïde, le cancer du poumon, le cancer des os, les myélomes multiples, les mésothéliomes et les mélanomes, le cancer de la peau, le cancer du sein, le cancer de la prostate, le cancer de la vessie, le cancer de l'utérus, le cancer des testicules, les lymphomes non-Hodgkinien, la leucémie, la maladie de Hodgkin, et des cancers des tissus mous, ainsi que des localisations secondaires métastatiques des cancers cités précédemment.

## Claims

1. Use of a compound corresponding to the following formula (I), or one of its pharmaceutically acceptable salts, in the preparation of a composition intended to prevent or treat at least one disease involving an abnormal cell proliferation: 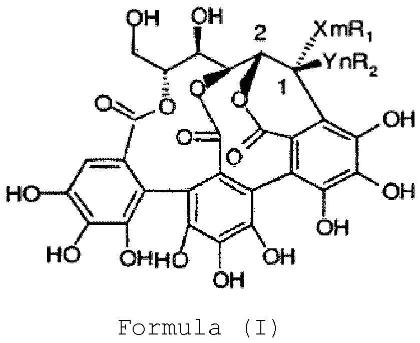 in which:
- XₘR₁ and YₙR₂, which are identical or different, are present, or
- one of XₘR₁ or YₙR₂ is absent and, in this case, the bond between C₁ and C₂ is a double bond,
- XₘR₁ and YₙR₂ together represent a group A and, in this case, the bond between C₁ and A is a double bond,
where:
- R₁ and R₂ represent, independently of one another, a hydrogen atom; a halogen atom; an alkyl, alkenyl or alkynyl radical comprising from 1 to 18 carbon atoms, optionally substituted by one or more amino, acid, acid derivative, alkoxy, aryl or hydroxyl groups; an aryl radical, optionally substituted by one or more amino, acid, acid derivative, alkoxy, aryl or hydroxyl groups; an arylalkyl or alkylaryl radical, optionally substituted by one or more amino, acid, acid derivative, alkoxy, aryl or hydroxyl groups; or a nitro functional group;
or R₁ and R₂ together form a ring or a heterocycle which is optionally substituted,
- m is an integer chosen from 0 and 1 and n is an integer chosen from 0 and 1,
- X and Y represent, independently of one another, -0-, -OC(=O)-, -OC(=O)O-, -OC(=O)N-, -NR₃-, -NR₃C(=O)-, -NR₃C(=O)O-, -NR₃C(O)NR₃-, -C(=O)-, -C(=O)O-, -S(O)_{q}- or -P(O)ᵣ-,
- q is an integer chosen from 0, 1, 2 and 3 and r is an integer chosen from 0, 1, 2 and 3,
- R₃ represents a hydrogen atom; an alkyl, alkenyl or alkynyl radical comprising 1 to 18 carbons, optionally substituted by one or more amino, acid, acid derivative, alkoxy, aryl or hydroxyl groups; an aryl radical, optionally substituted by one or more amino, acid, acid derivative, alkoxy, aryl or hydroxyl groups; an arylalkyl or alkylaryl radical, optionally substituted by one or more amino, acid, acid derivative, alkoxy, aryl or hydroxyl groups; or an alkoxy radical, optionally substituted by an aryl radical.
- A represents 0, S, NOR₅, NR₆ or CR₅R₆, where R₅ and R₆ represent, independently of one another, a hydrogen atom; an alkyl, alkenyl or alkynyl radical comprising from 1 to 18 carbon atoms, optionally substituted by one or more amino, acid, acid derivative, alkoxy, aryl or hydroxyl groups; an arylalkyl or alkylaryl radical, optionally substituted by one or more amino, acid, acid derivative, alkoxy, aryl or hydroxyl groups; or an alkoxy radical, optionally substituted by an aryl radical.

2. Use according to Claim 1, **characterized in that** the said compound corresponds to the following formula (I) : 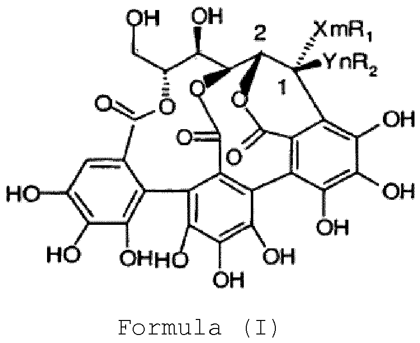 in which:
- R₁ and R₂ represent, independently of one another, a hydrogen atom; a halogen atom; an alkyl, alkenyl or alkynyl radical comprising from 1 to 18 carbon atoms, optionally substituted by one or more amino, acid, acid derivative, alkoxy, aryl or hydroxyl groups; an aryl radical, optionally substituted by one or more amino, acid, acid derivative, alkoxy, aryl or hydroxyl groups; an arylalkyl or alkylaryl radical, optionally substituted by one or more amino, acid, acid derivative, alkoxy, aryl or hydroxyl groups; or a nitro functional group;
or R₁ and R₂ together form a ring or a heterocycle which is optionally substituted,
- m is an integer chosen from 0 and 1 and n is an integer chosen from 0 and 1,
- X and Y represent, independently of one another, -0-, -OC(=O)-, -OC(=O)O-, -OC(=O)N-, -NR₃-, -NR₃C(=O)-, -NR₃C(=O)O-, -NR₃C(O)NR₃-, -C(=O)-, -C(=O)O-, -S(O)_{q}- or -P(O)ᵣ-,
- q is an integer chosen from 0, 1, 2 and 3 and r is an integer chosen from 0, 1, 2 and 3,
- R₃ represents a hydrogen atom; an alkyl, alkenyl or alkynyl radical comprising 1 to 18 carbons, optionally substituted by one or more amino, acid, acid derivative, alkoxy, aryl or hydroxyl groups; an aryl radical, optionally substituted by one or more amino, acid, acid derivative, alkoxy, aryl or hydroxyl groups; an arylalkyl or alkylaryl radical, optionally substituted by one or more amino, acid, acid derivative, alkoxy, aryl or hydroxyl groups; or an alkoxy radical, optionally substituted by an aryl radical.

3. Use according to Claim 2, **characterized in that** the said compound corresponds to the formula (I) in which m is 0 and R₁ represents a hydrogen atom, n is 1, Y represents -0- and R₂ represents a group chosen from the group comprising a hydrogen atom and an alkyl radical comprising from 1 to 18 carbon atoms.

4. Use according to Claim 2, **characterized in that** the said compound corresponds to the formula (I) in which m is 1, X represents -0-, R₁ represents a group chosen from the group consisting of a hydrogen atom and an alkyl radical comprising from 1 to 18 carbon atoms, n is 0 and R₂ represents a hydrogen atom.

5. Use according to Claim 1, **characterized in that** the said compound corresponds to the following formula (II): 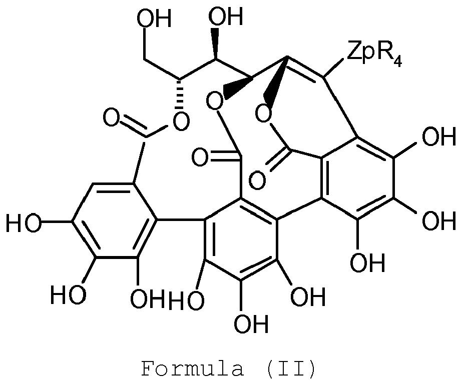 in which:
- p represents an integer chosen from 0 and 1, Z represents -0-, -OC(=O)-, -OC(=O)O-, -OC(=O)N-, -NR₃-, -NR₃C(=O)-, -NR₃C(=O)O-, -NR₃C(O)NR₃-, -C(=O)-, -C(=O)O-, -S(O)_{q}- or -P(O)ᵣ-,
- q is an integer chosen from 0, 1, 2 and 3 and r is an integer chosen from 0, 1, 2 and 3,
- R₃ represents a hydrogen atom; an alkyl, alkenyl or alkynyl radical comprising from 1 to 18 carbons, optionally substituted by one or more amino, acid, acid derivative, alkoxy, aryl or hydroxyl groups; an arylalkyl or alkylaryl radical, optionally substituted by one or more amino, acid, acid derivative, alkoxy, aryl or hydroxyl groups; or an alkoxy radical, optionally substituted by an aryl radical, and
- R₄ represents a hydrogen atom; a halogen atom; an alkyl, alkenyl or alkynyl radical comprising 1 to 18 carbon atoms, optionally substituted by one or more amino, acid, acid derivative, alkoxy, aryl or hydroxyl groups; an aryl radical, optionally substituted by one or more amino, acid, acid derivative, alkoxy, aryl or hydroxyl groups; an arylalkyl or alkylaryl radical, optionally substituted by one or more amino, acid, acid derivative, alkoxy, aryl or hydroxyl groups; an alkoxy radical, optionally substituted by an aryl radical; or a nitro functional group.

6. Use according to Claim 5, **characterized in that** the said compound corresponds to the formula (II) in which p is 0 and R₄ represents a group chosen from the group consisting of a hydrogen atom and an alkyl radical comprising from 1 to 18 carbon atoms.

7. Use according to Claim 1, **characterized in that** the said compound corresponds to the following formula (III): 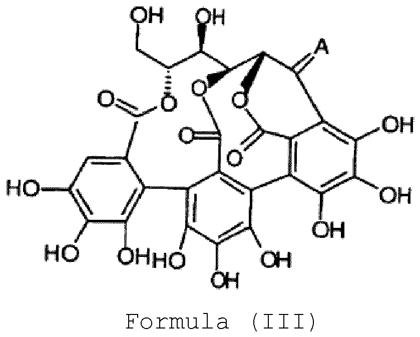 in which:
- A represents 0, S, NOR₅, NR₆ or CR₅R₆,
- R₅ represents a hydrogen atom; an alkyl, alkenyl or alkynyl radical comprising from 1 to 18 carbon atoms, optionally substituted by one or more amino, acid, acid derivative, alkoxy, aryl or hydroxyl groups; an aryl radical, optionally substituted by one or more amino, acid, acid derivative, alkoxy, aryl or hydroxyl groups; an arylalkyl or alkylaryl radical, optionally substituted by one or more amino, acid, acid derivative, alkoxy, aryl or hydroxyl groups; or an alkoxy radical, optionally substituted by an aryl radical, and
- R₆ represents an alkyl, alkenyl or alkynyl radical comprising 1 to 18 carbon atoms, optionally substituted by one or more amino, acid, acid derivative, alkoxy, aryl or hydroxyl groups; an aryl radical, optionally substituted by one or more amino, acid, acid derivative, alkoxy, aryl or hydroxyl groups; an arylalkyl or alkylaryl radical, optionally substituted by one or more amino, acid, acid derivative, alkoxy, aryl or hydroxyl groups; or an alkoxy radical, optionally substituted by an aryl radical.

8. Use according to Claim 1, **characterized in that** the said compound corresponds to the formula (III) in which A represents O.

9. Use according to any one of Claims 1 to 8, in which the said disease involving an abnormal cell proliferation is cancer.

10. Use according to Claim 9, in which the said cancer is a cancer chosen from pancreatic cancer, oropharyngeal cancer, stomach cancer, oesophageal cancer, colorectal cancer, brain cancer, in particular gliomas, ovarian cancer, liver cancer, kidney cancer, laryngeal cancer, thyroid cancer, lung cancer, bone cancer, multiple myelomas, mesotheliomas and melanomas, skin cancer, breast cancer, prostate cancer, bladder cancer, uterine cancer, testicular cancer, non-Hodgkin's lymphoma, leukaemia, Hodgkin's disease and soft tissue cancers, as well as secondary metastatic occurrences of the abovementioned cancers.

11. Use according to any one of Claims 1 to 8, in which the said composition intended to prevent or treat at least one disease involving abnormal cell proliferation is a pro-apoptotic composition.

12. Compound as defined according to any one of Claims 1 to 8, for the prevention or treatment of at least one disease involving abnormal cell proliferation.

13. Compound according to Claim 12, **characterized in that** the said disease involving abnormal cell proliferation is cancer.

14. Compound according to Claim 13, **characterized in that** the said cancer is a cancer chosen from pancreatic cancer, oropharyngeal cancer, stomach cancer, oesophageal cancer, colorectal cancer, brain cancer, in particular gliomas, ovarian cancer, liver cancer, kidney cancer, laryngeal cancer, thyroid cancer, lung cancer, bone cancer, multiple myelomas, mesotheliomas and melanomas, skin cancer, breast cancer, prostate cancer, bladder cancer, uterine cancer, testicular cancer, non-Hodgkin's lymphoma, leukaemia, Hodgkin's disease and soft tissue cancers, as well as secondary metastatic occurrences of the abovementioned cancers.

## Patentansprüche

1. Verwendung einer Verbindung oder eines ihrer pharmazeutisch akzeptablen Salze gemäß nachstehender Formel (I), bei der Herstellung einer Zusammensetzung zur Vorbeugung oder Behandlung mindestens einer Erkrankung mit abnormaler Zellproliferation : 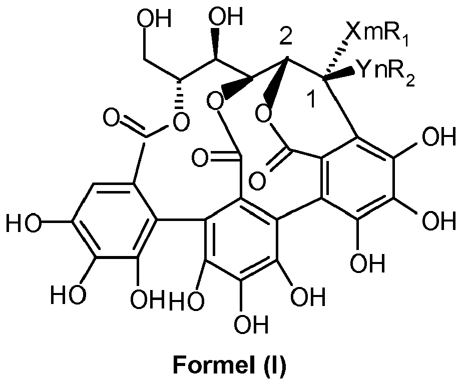 In welcher :
- identische oder unterschiedliche XₘR₁ und YₙR₂, vorhanden sind, oder
- entweder XₘR₁ oder YₙR₂ fehlt, und in diesem Fall die Verbindung zwischen C₁ und C₂ eine Doppelbindung ist,
- XₘR₁ und YₙR₂ zusammen eine Gruppe A darstellen und in diesem Fall die Verbindung zwischen C₁ und A eine Doppelbindung ist,
wobei:
- R₁ und R₂ unabhängig voneinander ein Wasserstoffatom ; ein Halogenatom; ein Alkyl-, Alken-oder Alkinradikal mit 1 bis 18 Kohlenstoffatomen darstellen, gegebenenfalls durch ein oder mehrere Amino-, Säuren-, Säurenderivat, Alkoxy-, Aryl- oder Hydroxygruppen; ein Arylradikal ersetzt, gegebenenfalls ersetzt durch ein oder mehrere Amino-, Säuren-, Säurenderivat, Alkoxy-, Aryl- oder Hydroxygruppen; ein Arylalkyl- oder Alkylarylradikal ersetzt, gegebenenfalls ersetzt durch ein oder mehrere Amino-, Säuren-, Säurenderivat, Alkoxy-, Aryl- oder Hydroxygruppen; oder eine Nitrofunktion ersetzt;
oder R₁ und R₂ gemeinsam einen Cyklus oder einen Heterocyklus bilden, gegebenenfalls ersetzt,
- m eine ganze zwischen 0 und 1 gewählte Zahl ist, n eine ganze zwischen 0 und 1 gewählte Zahl ist,
- X und Y unabhängig voneinander -O-, OC(=O)-, -OC(=O)O-, -OC(=O)N-, -NR₃-, -NR₃C(=O)-, NR₃C(=O)O-, -NR₃C(O)NR₃-, -C(=O)-, -C(=O)O-, -S(O)_{q}-, oder -P(O)ᵣ- darstellen,
- q eine ganze zwischen 0, 1, 2 und 3 gewählte Zahl ist, r eine ganze zwischen 0, 1, 2 und 3 gewählte Zahl ist,
- R₃ ein Wasserstoffatom; ein Alkyl-, Alken- oder Alkinradikal mit 1 bis 18 Kohlenstoffatomen darstellt , gegebenenfalls durch ein oder mehrere Amino-, Säuren-, Säurenderivat, Alkoxy-, Aryl- oder Hydroxygruppen; ein Arylradikal ersetzt, gegebenenfalls durch ein oder mehrere Amino-, Säuren-, Säurenderivat, Alkoxy-, Aryl- oder Hydroxygruppen; ein Arylalkyl- oder Alkylarylradikal ersetzt, gegebenenfalls durch ein oder mehrere Amino-, Säuren-, Säurenderivat, Alkoxy-, Aryl- oder Hydroxygruppen; oder ein Alkoxyradikal ersetzt, gegebenenfalls durch ein Arylradikal ersetzt;
- A stellt 0, S, NOR₅, NR₆ oder CR₅R₆ dar, wobei R₅ und R₆ unabhängig voneinander ein Wasserstoffatom; ein Alkyl- Alken- oder Alkinradikal, mit 1 bis 18 Kohlenstoffatomen darstellen, gegebenenfalls durch eine oder mehrere Amino-, Säuren-, Säurenderivat-, Alkoxy-, Aryl- oder Hydroxygruppen; ein Arylalkyl- oder Alkylarylradiakal ersetzt, gegebenenfalls durch ein oder mehrere Amino-, Säuren-, Säurenderivat-, Alkoxy-, Aryl- oder Hydroxygruppen; oder ein Alkoyxradikal ersetzt, gegebenenfalls durch ein Arylradikal ersetzt.

2. Verwendung nach Anspruch 1, **dadurch gekennzeichnet, dass** die besagte Verbindung folgender Formel (I) entspricht : 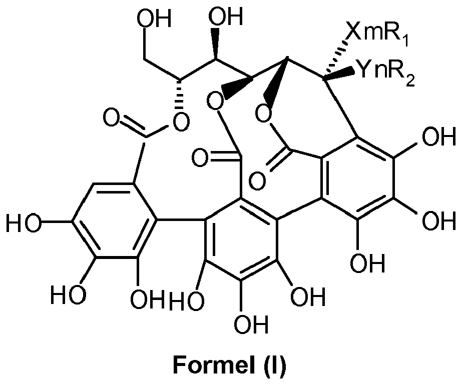 In welcher :
- R₁ und R₂ unabhängig voneinander ein Wasserstoffatom, ein Halogenatom; ein Alkyl-, Alken-oder Alkinradikal, mit 1 bis 18 Kahlenstoffatomen darstellen, gegebenenfalls durch ein oder mehrere Amino-, Säure-, Säurederivat-, Alkoxy-, Aryl- oder Hydroxygruppen; ein Arylradikal ersetzt, gegebenenfalls durch ein oder mehrere Amino-, Säure-, Säurederivat-, Alkoxy-, Aryl- oder Hydroxygruppen ; ein Arylalkyl- oder Alkylaryl ersetzt, gegebenenfalls durch eine Amino-, Säure-, Säurederivat-, Alkoxy-, Aryl- oder Hydroxygruppen; oder eine Nitrofunktion ersetzt;
oder R₁ und R₂ gemeinsam einen Cyklus oder Heterocyklus bilden, gegebenenfalls ersetzt,
- m eine ganze zwischen 0 und 1 gewählte Zahl ist, n eine ganze zwischen 0 und 1 gewählte Zahl ist,
- X und Y unabhängig voneinander -O-, OC(=O)-, -OC(=O)O-, -OC(=O)N-, -NR₃-, -NR₃C(=O)-, NR₃C(=O)O-, -NR₃C(O)NR₃-, -C(=O)-, -C(=O)O-, -S(O)_{q}-, ou -P(O)ᵣ- darstellen,
- q eine ganze zwischen 0, 1, 2 und 3 gewählte Zahl ist, r eine ganze zwischen 0, 1, 2 und 3 gewählte Zahl ist,
- R₃ ein Wasserstoffatom; ein Alkyl-, Alken- oder Alkinradikal mit 1 bis 18 Kohlenstoffatomen darstellt , gegebenenfalls durch ein oder mehrere Amino-, Säuren-, Säurenderivat, Alkoxy-, Aryl- oder Hydroxygruppen; ein Arylalkyl- oder Alkylarylradiakal ersetzt, gegebenenfalls durch ein oder mehrere Amino-, Säuren-, Säurenderivat, Alkoxy-, Aryl- oder Hydroxygruppen; ein Alkoxyradikal ersetzt, gegebenenfalls durch ein Arylradikal ersetzt.

3. Verwendung nach Anspruch 2, **dadurch gekennzeichnet, dass** besagte Verbindung der Formel (I) entspricht, in welcher m gleich 0 ist und R₁ ein Wasserstoffatom darstellt, n gleich 1 ist, Y -O-darstellt und R₂ eine Gruppe darstellt, die innerhalb der Gruppe gewählt wird, die ein Wasserstoffatom und ein Alkylradikal mit 1 bis 18 Kohlenstoffatomen umfasst.

4. Verwendung nach Anspruch 2, **dadurch gekennzeichnet, dass** besagte Verbindung der Formel (I) entspricht, in welcher m gleich 1 ist, X -0- darstellt, R₁ eine Gruppe darstellt, die innerhalb der Gruppe gewählt wird, die ein Wasserstoffatom und ein Alkylradikal mit 1 bis 18 Kohlenstoffatomen umfasst, n gleich 0 ist, und R₂ ein Wasserstoffatom darstellt.

5. Verwendung nach Anspruch 1, **dadurch gekennzeichnet, dass** besagte Verbindung der folgenden Formel (II) entspricht : 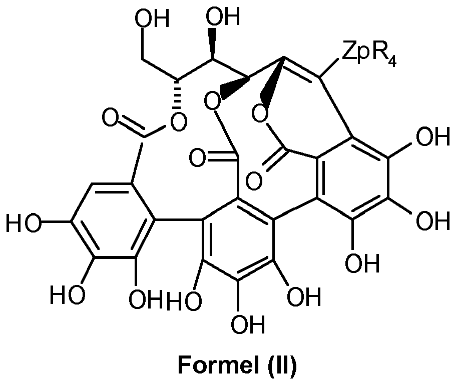 In welcher :
- p eine ganze zwischen 0 und 1 gewählte Zahl ist,
- Z -O-, -OC(=O)-, -OC(=O)O-, -OC(=O)N-, - NR₃-, -NR₃C(=O)-, -NR₃C(=O)O-, -NR₃C(O)NR₃-, -C(=O)-, - C(=O)O-, -S(O)_{q}-, oder -P(O)ᵣ- darstellt,
- q eine ganze zwischen 0, 1, 2 und 3 gewählte Zahl ist, r eine ganze zwischen 0, 1, 2 und 3 gewählte Zahl ist,
- R₃ ein Wasserstoffatom; ein Alkyl-, Alken- oder Alkinradikal mit 1 bis 18 Kohlenstoffatomen darstellt , gegebenenfalls durch ein oder mehrere Amino-, Säuren-, Säurenderivat, Alkoxy-, Aryl- oder Hydroxygruppen; ein Arylalkyl- oder Alkylarylradiakal ersetzt, gegebenenfalls durch ein oder mehrere Amino-, Säuren-, Säurenderivat, Alkoxy-, Aryl- oder Hydroxy-gruppen; oder ein Alkoxyradiakal ersetzt, gegebenenfalls durch ein Arylradikal ersetzt, und
- R₄ ein Wasserstoffatom; ein Halogenatom; ein Alkyl-, Alken- oder Alkinradikal, mit 1 bis 18 Kohlenstoffatomen darstellt, gegebenenfalls durch ein oder mehrere Amino-, Säure-, Säurederivat-, Alkoxy-, Aryl- oder Hydroxygruppen; ein Arylradikal ersetzt, gegebenenfalls durch ein oder mehrere Amino-, Säure-, Säurederivat-, Alkoxy-, Aryl- oder Hydroxygruppen ; ein Arylaklyl- oder Alkylarylradikal ersetzt, gegebenenfalls durch ein oder mehrere Amino-, Säure-, Säurederivat-, Alkoxy-, Aryl- oder Hydroxygruppen; ein Alkoxyradikal ersetzt, gegebenenfalls ein Arylradikal; oder eine Nitrofunktion ersetzt.

6. Verwendung nach Anspruch 5, **dadurch gekennzeichnet, dass** besagte Verbindung der Formel (II) entspricht, in welcher p gleich 0 ist und R₄ eine Gruppe darstellt, die innerhalb der Gruppe gewählt wird, die ein Wasserstoffatom und ein Alkylradikal mit 1 bis 18 Kohlenstoffatomen umfasst.

7. Verwendung nach Anspruch 1, **dadurch gekennzeichnet, dass** besagte Verbindung der folgenden Formel (III) entspricht : 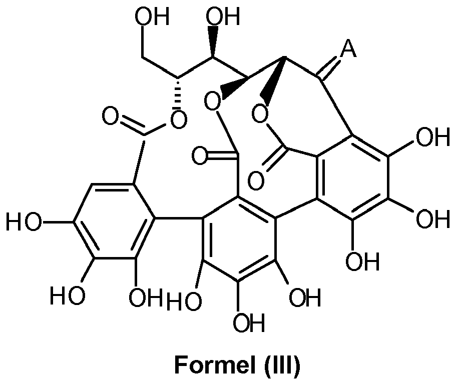 In welcher :
- A 0, S, NOR₅, NR₆ oder CR₅R₆ darstellt,
- R₅ ein Wasserstoffatom; ein Alkyl-, Alken- oder Alkinradikal mit 1 bis 18 Kohlenstoffatomen darstellt , gegebenenfalls durch ein oder mehrere Amino-, Säuren-, Säurenderivat, Alkoxy-, Aryl- oder Hydroxygruppen; ein Arylradikal ersetzt, gegebenenfalls durch ein oder mehrere Amino-, Säuren-, Säurenderivat, Alkoxy-, Aryl- oder Hydroxygruppen; ein Arylalkyl- oder Alkylarylradiakal ersetzt, gegebenenfalls durch ein oder mehrere Amino-, Säuren-, Säurenderivat, Alkoxy-, Aryl- oder Hydroxygruppen; ein Alkoxyradikal ersetzt, gegebenenfalls durch ein Arylradikal ersetzt, und
- R₆ ein Alkyl-, Alken- oder Alkinradikal mit 1 bis 18 Kohlenstoffatomen darstellt, gegebenenfalls durch ein oder mehrere Amino-, Säuren-, Säurenderivat, Alkoxy-, Aryl- oder Hydroxygruppen; ein Arylradikal ersetzt, gegebenenfalls durch ein oder mehrere Amino-, Säuren-, Säurenderivat, Alkoxy-, Aryl- oder Hydroxygruppen; ein Arylalkyl- oder Alkylarylradiakal ersetzt, gegebenenfalls durch ein oder mehrere Amino-, Säuren-, Säurenderivat, Alkoxy-, Aryl- oder Hydroxygruppen; ein Alkoxyradikal ersetzt, gegebenenfalls durch ein Arylradikal ersetzt.

8. Verwendung nach Anspruch 1, **dadurch gekennzeichnet, dass** besagte Verbindung der Formel (III) entspricht, in welcher A O darstellt.

9. Verwendung nach einem der Ansprüche 1 bis 8 in welcher besagte Erkrankung mit abnormaler Zellproliferation Krebs ist.

10. Verwendung nach Anspruch 9, wobei besagter Krebs ein Krebs unter folgenden ist: Bauchspeicheldrüsenkrebs, Mundhöhle- und Rachenkrebs, Magenkrebs, Speiseröhrenkrebs, Kolon- und Rektalkrebs, Hirnkrebs, insbesondere der Gliome, Eierstockkrebs, Leberkrebs, Nierenkrebs, Kehlkopfkrebs, Schilddrüsenkrebs, Lungenkrebs, Knochenkrebs, multiple Myelome, Mesotheliome und Melanome, Hautkrebs, Brustkrebs, Prostatakrebs, Blasenkrebs, Gebärmutterkrebs, Hodenkrebs, Non-Hodgkin-Lymphome, Leukämie, Morbus Hodgkin, und Weichteilkrebs, sowie sekundäre metastatische Lokalisationen der vorgenannten Krebsarten.

11. Verwendung nach einem der Ansprüche 1 bis 8, in welcher besagte Verbindung zur Vorbeugung oder Behandlung mindestens einer Erkrankung mit abnormaler Zellproliferation eine pro-apoptotische Verbindung ist.

12. Zusammensetzung gemäß der Definition in einem der Ansprüche 1 bis 8 zur Vorbeugung oder Behandlung mindestens Erkrankung mit abnormaler Zellproliferation auftritt.

13. Zusammensetzung nach Anspruch 12, **dadurch gekennzeichnet, dass** besagte Erkrankung mit abnormaler Zellproliferation Krebs ist.

14. Zusammensetzung nach Anspruch 13, **dadurch gekennzeichnet, dass** besagter Krebs einer unter folgenden ist : Bauchspeicheldrüsenkrebs, Mundhöhle-und Rachenkrebs, Magenkrebs, Speiseröhren-krebs, Kolon-und Rektalkrebs, Hirnkrebs, insbesondere der Gliome, Eierstockkrebs, Leberkrebs, Nierenkrebs, Kehlkopfkrebs, Schilddrüsenkrebs, Lungenkrebs, Knochenkrebs, multiple Myelome, Mesotheliome et Melanome, Hautkrebs, Brustkrebs, Prostatakrebs, Blasenkrebs, Gebärmutterkrebs, Hodenkrebs, Non-Hodgkin-Lymphome, Leukämie, Morbus Hodgkin, und Weichteilkrebs, sowie sekundäre metastatische Lokalisationen der vorgenannten Krebsarten.
